Europäisches Patentamt

European Patent Office

Office européen des brevets

(19)

(11) **EP 0 678 337 B1**

(12)         **EUROPEAN PATENT SPECIFICATION**

(45) Date of publication and mention
of the grant of the patent:
**23.01.2002   Bulletin 2002/04**

(51) Int Cl.7: **B05B 17/06**, B05B 5/025

(21) Application number: **95104175.5**

(22) Date of filing: **22.03.1995**

(54) **Apparatus**

Vorrichtung

Appareil

(84) Designated Contracting States:
**AT BE CH DE DK ES FR GB GR IE IT LI LU MC NL
PT SE**

(30) Priority: **25.03.1994   GB 9405952**

(43) Date of publication of application:
**25.10.1995   Bulletin 1995/43**

(73) Proprietor: **AstraZeneca AB
151 85 Södertälje (SE)**

(72) Inventor: **Rowe, Raymond Charles
Macclesfield, Cheshire SK10 4TG (GB)**

(74) Representative: **Bryant, Tracey et al
AstraZeneca PLC Global Intellectual Property
Mereside Alderley Park
Macclesfield, Cheshire SK10 4TG (GB)**

(56) References cited:
**EP-A- 0 011 269         EP-A- 0 224 352
EP-A- 0 389 665         EP-A- 0 590 165
WO-A-85/00761         FR-A- 1 271 341**

• **REVIEW OF SCIENTIFIC INSTRUMENTS, no.6,
June 1990, NEW YORK, US pages 1689 - 1693,
XP166168 KYUN-JIN CHOI AND BRAIN
DELCORIO 'GENERATION OF CONTROLLABLE
MONODISPERSED SPRAYS USING IMPULSE
JET AND CHARGING TECHNIQUES'**
• **F&M FEINWERKTECHNIK & MESSTECHNIK,
vol.27, no.11, November 1991, MÜNCHEN, DE
pages 459 - 463, XP274115 M. DÖRING
'FLÜSSIGKEITTEN MIKROFEIN DOSIEREN'**

**Description**

This invention relates to apparatus suitable for the delivery of sprays of aqueous solutions or suspensions to the eye.

A conventional method of ocular administration of aqueous solutions or suspensions comprises the use of eye drops. This is generally known to have low patient acceptability, especially in the young, and it is necessary, for administration, to incline the recipient's head towards a horizontal position. The administration of a large drop of liquid to the eye initiates a blink reflex, which can result in a substantial wastage of the applied liquid or suspension by drainage either through the tear ducts or onto the skin surface. Indeed, it has been reported that if a 30-50µl drop is applied to the eye, the actual volume that remains at the target is only 5-7µl. Therefore, in addition to the low patient acceptability, there is a 4- to 10-fold wastage. This leads to inefficiency in the use of expensive ingredients and, in addition, the administrator has little control, and is uncertain, over the amount of liquid which actually reaches the target. This is particularly important if the liquid is a solution or suspension of an ophthalmologically-active therapeutic substance.

Another conventional method of ocular administration of an ophthalmologically-active therapeutic substance comprises the use of an ointment. This similarly has been found to have low patient acceptability and, in this method also, a substantial wastage of active ingredient can result.

These problems in the efficient administration of therapeutically active substances to the eye are largely overcome in European Patent No. 0 224 352B by generating a spray of electrically charged droplets of a liquid formulation comprising an ophthalmologically-active substance and an ophthalmologically-acceptable diluent, for subsequent administration to the eye.

A related piece of apparatus is discussed by K Choi and B Delcorio in 'Review of Scientific Instruments', Vol 61, No. 6, pp 1689-1693. This discloses an apparatus for producing a tunable monodispersable spray in which a single orifice impulse -jet droplet generator is used to produce a single stream of uniform droplets. The single stream of droplets is dispersed by electrostatic charging.

The formulation of EP 224 352 has a viscosity in the range $10^{-3}$ to 1.0Pa.s at 25°C, and a resistivity in the range $10^4$ to $10^{12}$ohm.cm at 25°C. The formulation is applied to a spray nozzle wherein a sufficiently large electrical potential relative to earth is applied to the formulation from a high voltage generator, that sufficient electrical gradient is produced at the nozzle to atomize the formulation as a spray of electrically charged droplets.

Although such a method allows the delivery to the eye of an optimum small volume of a formulation of a therapeutic substance, without requi-ring the recipient's head to be inclined towards the horizontal, it does, however, still have some drawbacks. Solutions or suspensions containing more than about 50% of water, that is, of lower resistivity than $10^4$ohm.cm, cannot be sprayed, and high voltages of 15kV or higher are used. Further, an electrode needs to be in contact with the formulation, to achieve the correct voltage for atomization, and this could cause cross-contamination problems for pharmaceutical formulations. A further disadvantage is that a formulation containing substantial amounts of non-aqueous solvents, which is to be dispensed by this method, is likely to be hypertonic, which although acceptable for very low volume applications can result in a stinging sensation if larger volumes are administered to the eye.

The present invention provides an apparatus for accurate dispensing of a low volume of a solution or suspension to the eye without the above-mentioned drawbacks. In particular the present invention provides apparatus which allows the dispensing of isotonic solutions, which avoids stinging sensations, it allows the use of suspensions as well as solutions. A further advantage is that the apparatus enables the use of formulations with reduced levels of non-aqueous solvents. This is achieved by an apparatus in a which a colinear stream of uniformly-sized, equally spaced droplets of a liquid formulation are produced, using either piezoelectric or electromagnetic transducers to cause uniform break-up of a jet of the formulation emitted from a nozzle. The droplets so produced are initially not electrically charged, and charging can be accomplished subsequently by passing the stream of droplets through a cylindrical charging electrode longitudinally positioned so that induced electric charges are trapped on the droplets as they pass through the cylindrical electrode.

As indicated above, conventional methods for ocular administration lead to wastage of ingredient, for example by drainage through the naso-lachrymal duct into the throat, and subsequent ingestion into the gastro-intestinal tract, whence it can be absorbed systemically and exert undesired side-effects. For example, it is well documented in the literature that b-adrenoceptor antagonists administered as eye-drops can exert a significant cardiovascular effect as a result of such ingestion into the gastro-intestinal tract.

The present invention provides apparatus which enables accurate targetting of a fine spray of electrically charged droplets of a liquid formulation to dose just the required amount of an ophthalmologically active substance, thereby substantially eliminating unwanted side-effects.

Thus, according to the invention, there is provided

1. Spraying apparatus suitable for dispensing a liquid formulation to the eye comprises:

(i) at least one spray nozzle having an outlet of sufficiently small cross section to be capable of retaining an appropriate amount of a liquid formulation, by surface tension;

(ii) means to supply an appropriate measured volume of a liquid formulation to the spray nozzle;

(iii) means to eject a measured volume of liquid formulation from the spray nozzle as a jet;

(iv) a piezoelectric or electromagnetic transducer for exciting the jet of liquid formulation emitted from the spray nozzle to form a stream of droplets of liquid formulation;

(v) a charging electrode spaced co-axially in front of the spray nozzle and so spaced that the stream of droplets, immediately they are formed, are within the charging field of the electrode ; and

(vi) means for applying a voltage to the electrode ;

wherein at least the spray nozzle is suitable to be hand held when in use.

The liquid ophthalmic formulation may be a hygiene product, for example an eyewash or artificial tears for the treatment of dry eye, or a moistening or lubricating product for contact lens users, in the form of a conventional, predominantly aqueous and essentially isotonic liquid preparation, or it may be a product containing an ophthalmologically-active substance.

The ophthalmologically active substances encompassed by this invention are any compounds having a pharmacological effect on and/or in the eye. Typical of such compounds are chemotherapeutic agents, compounds to aid ocular examination, and compounds to aid surgery, for example:

(a) anti-inflammatory agents, such as prednisolone and other corticosteroids;

(b) antimicrobial drugs, such as antibiotics, antiseptics, antivirals, fungicides and sulphonamides, for example chloramphenicol, sulphacetamide, gentamycin, nystatin, acyclovir and idoxuridine;

(c) autonomic drugs, such as b-adrenoceptor antagonists, cycloplegics, miotics, mydriatics and vasoconstrictors, for example timolol, atenolol, pilocarpine, atropine, tropicamide, hyoscine, ephedrine, phenylephrine, carbachol, guanethidine and adrenaline;

(d) local anaesthetics, such a lignocaine or oxybuprocaine;

(e) diagnostics, such as fluorescein;

(f) drugs to assist healing of corneal abrasions, such as urogastrone and epidermal growth factor (EGF);

of which (c) is a particularly important group.

Suitably, the ophthalmologically active substance is present in the formulation in a concentration range of from about 0.1% to about 20%, and preferably from about 5% to about 10%, but the required concentration depends, naturally, upon the potency of the particular active substance being used.

A resistivity lower than $10^4$ohm.cm for the liquid ophthalmic formulation is preferred and this is achieved by making it predominantly aqueous, although a small proportion of non-aqueous liquids, up to about 20%, may also be incorporated. Suitable such non-aqueous liquids are, for example, glycerol, propylene glycol, polyethylene glycol of average molecular weight up to about 600, and dimethyl isosorbide.

The viscosity of the formulation for use with the apparatus according to the invention may be adjusted to within the required range by the addition of viscolysers, for example hydroxyethylcellulose, hydroxypropylcellulose, carboxymethylcellulose, hydroxypropylmethylcellulose, methylcellulose, polyvinyl alcohol, polyethylene glycol, dextran or polyvinylpyrrolidone.

The tonicity of such a formulation may be adjusted into the range tolerated by the eye, for example tonicity equivalent to 0.2-1.4% w/v sodium chloride, by the addition of a tonicity modifier. A preferred range of tonicity is equivalent to from 0.6-1.0% w/v sodium chloride, and especially preferred are solutions having a tonicity as close as possible to 0.9% w/v sodium chloride. A suitable tonicity modifier is, for example, sodium chloride itself. The addition of sodium chloride as a tonicity modifier also has the effect of lowering the resistivity of the formulation.

The formulation may also contain a preservative, for example benzalkonium chloride, chlorhexidine acetate, phenylmercuric acetate, phenylmercuric nitrate, thiomersal, chlorbutol, benzyl alcohol or p-hydroxybenzoates.

The formulation may also contain a pH buffer salt, to maintain the pH of the formulation at an optimum to minimize chemical degradation, to increase comfort for the user, and to enhance therapeutic effect. Suitable such buffer salts are, for example, borate buffer (boric acid/borax), phosphate buffer (sodium hydrogen phosphate/sodium phosphate) and citrate buffer (citric acid/sodium citrate).

Several drugs used in ophthalmic formulations oxidize on exposure to air, with loss of potency, and the formulation may therefore advantageously contain an antioxidant, for example sodium metabisulfite for acid formulations, or sodium sulfite for alkaline formulations.

A chelating agent, for example disodium edetate, may also be included, to remove traces of heavy metals, where the presence of such impurities catalyses the breakdown of the drug. Disodium edetate also has the effect of enhancing the activity of certain preservatives, and the concentration of benzalkonium chloride, for example, may be reduced when disodium edetate is also present in the formulation.

In one embodiment of this invention, the means to supply an appropriate measured volume of a liquid formulation is provided by a metered valve or syringe-pump of the type used for multi-dose administration of insulin, to control the passage of the liquid formulation from a reservoir in the apparatus, to the spray nozzle. Alternatively, accurately measured low volumes can be supplied to the apparatus by placing the spray nozzle in the liquid formulation and drawing in the required volume by pipette action, for example by using a piston in a syringe.

In a preferred aspect of this invention, we have found that the best spraying results are achieved using a modification of the previous apparatus, in which the spray nozzle is demountable from the apparatus. In use the required volume of formulation is placed in the demounted spray nozzle, which is then located on the spraying apparatus in any convenient manner, such as by screwing or by friction-fit on an appropriate receiving member. In this way, the low volume of formulation is measured in any convenient manner prior to use.

Piston action can also be used as the means to eject a measured volume of liquid formulation from the spray nozzle as a jet.

The means for exciting the the jet of liquid formulation emitted from the spray nozzle to form a stream of droplets of liquid formulation may, for example, a piezoelectric or an electromagnetic transducer. For optimum droplet generation, the jet of liquid formulation needs to be perturbed at a wavelength equal to 9.016 times the radius of the spray nozzle, so that, for example, for a nozzle of approximately 100μm diameter, frequencies of 1-200kHz, preferably 50-150kHz, are required.

The charging electrode which is spaced co-axially in front of the spray nozzle conveniently takes the form of a cylinder or annulus, co-axial with the spray nozzle, charged to a suitable positive or negative potential, but it may also be in the form of separate elements of any suitable form, located around the axis of the nozzle, and with a space between, through which the stream of droplets can be directed in order to acquire an electrical charge. As indicated above, for efficient use of the formulation, that is, so that all of the active ingredient reaches the treatment site, it is necessary that each droplet in the stream becomes electrically charged as soon as it is formed from the jet, so the charging electrode must be located in front of the nozzle so that immediately a droplet is formed, it is within the charging field of the charging electrode.

The resistivity of the liquid formulation must be chosen to be low enough to ensure that the droplets become fully charged within the duration of the charging electrode pulse, which will typically be 2-4μs. It can be shown mathematically that, for a given geometry, the charge on the droplets is determined solely by the voltage applied to the charging electrode. For droplets of approximately 100μm diameter, charging voltages in the range of about 0.1 to about 1000V are suitable, modulated at the same frequency as the drop generation rate, that is, the transducer frequency, or some sub-harmonic of it. Resistivities for the liquid formulation of less than $10^4$ohm.cm, and preferably in the range of $10^2$ to $10^3$ohm.cm, are required in order for the droplets to become fully charged.

Generally, in the apparatus of this invention comprises one or two spray nozzles, depending upon whether it is desired to treat eyes separately or concurrently. Conveniently, the voltage required to charge the charging electrode is provided by a battery powered voltage generator, housed in hand-held apparatus. In another embodiment, the voltage can be generated in a remote pack, and supplied by an electrical connection to a hand-held spraying apparatus. In another embodiment, the reservoir supplying the formulation to the spray nozzle may be remote from the hand-held spray nozzle, and connected thereto by appropriate flexible tubing. In another embodiment, both the voltage generator and the reservoir may be remote from the hand-held spray nozzle.

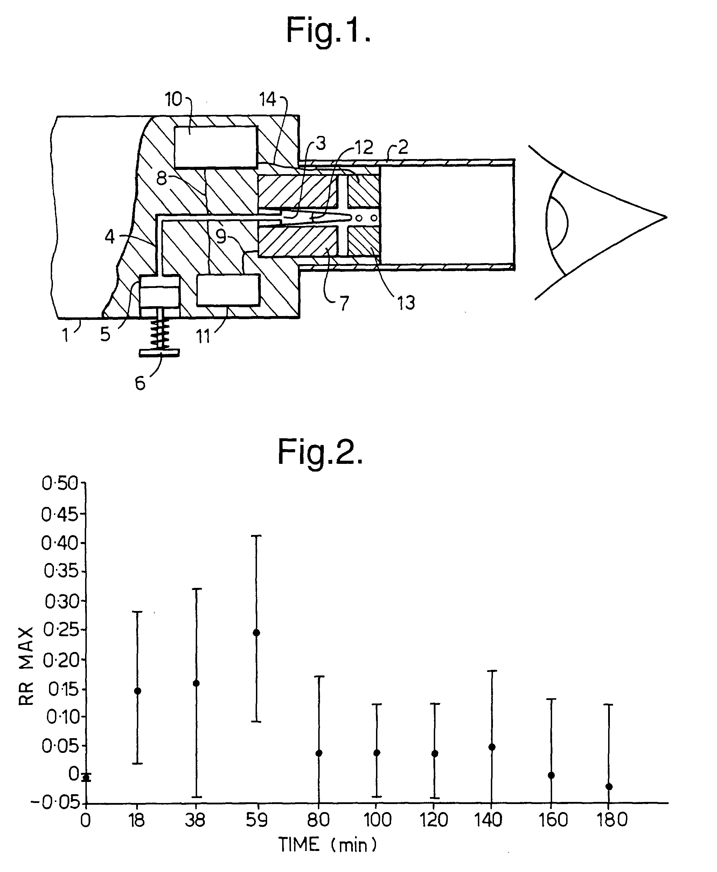

A particular embodiment will now be described, by way of example only, with reference to Figure 1, which is a schematic view illustrating the principal components of one form of the apparatus.

Referring to Figure 1, there is a body member 1 of a suitable size to be hand-held. On one wall of the body member 1 there is mounted a guide 2 in the form of a short tube, the diameter of which approximates to the size of the eye to be treated. In the portion of the wall of the body member 1 which lies within the guide tube 2 there is positioned centrally the outlet 3 of a narrow bore tube 4 which at its other end broadens to provide a wider bore section 5, positioned in a side wall of the body member 1, and in which a spring-loaded piston 6 can operate. Located in the wall of the body member 1, and disposed around the outlet 3 of the narrow bore tube 4 in an annular manner is a piezoelectric or electromagnetic transducer 7, which is powered through electrical connections 8 and 9 from a voltage generator 10 via a frequency control unit 11, both of which are housed within the body member 1. A demountable nozzle 12 is provided which is capable of being detached from the apparatus in order to be charged with the formulation to be administered, and then re-attached securely within the central orifice of the transducer, by a tight push fit, to cooperate with the outlet 3 of the narrow bore tube 4. Within the guide tube 2, adjacent to the transducer 7 and separated therefrom by a small distance, is an annular or cylindrical charging electrode 13, located co-axially with the transducer 7, and electrically insulated from the other parts of the apparatus. The charging electrode 13 is charged to suitable positive or negative potential through an electrical connection 14 from the voltage generator 10, and its pulse frequency is controlled through the frequency control unit 11.

In use, the demountable nozzle 12 is filled with the appropriate volume of the formulation to be administered, the apparatus is held in the hand, and the guide tube is positioned directed towards the eye to be treated, and a short distance therefrom. The voltage generator 10 is switched on, to activate the transducer 7 and the charging electrode 13. The piston 6 is then depressed against its spring, to create sufficient pressure within the narrow bore tube 4 to expel the liquid formulation from the nozzle 12, through the central orifice of the transducer 7, whereby the jet of liquid formulation is broken up to produce a colinear stream of uniformly sized, equally spaced, uncharged droplets. The stream of droplets then passes through the bore of the annular charging electrode 13, where each droplet acquires an electric charge. The frequency control unit 11 is pre-adjusted to ensure that the voltage on the charge electrode is varied at the same frequency as, or a greater frequency than, that at which the droplets are produced, so that each droplet is individually electrically charged. The charged droplets then continue towards the eye to be treated, where they discharge their electric charge at the first earthed surface they encounter, namely the tissue of the eyeball, to give the eyeball an even coating of the formulation, without sensation.

The invention will now be illustrated, but not limited, by the following Example:

Example 1

Ephedrine hydrochloride (0.25mg) was formulated as a 5% solution in physiological saline, radiolabelled with approximately 0.8Gbq of 99mTc-DTPA complex. This formulation was sprayed, from apparatus essentially as hereinbefore specdescribed in this specification, onto one eye of each of 6 New Zealand white rabbits, according to the following procedure:

The test animals were acclimatised to the experimental conditions, by exposure to constant light intensity and minimal distractions, for 20 minutes. They were then placed in restraining boxes which were positioned approximately 30-40cm in front of a camera (Pentax ME Super 35mm camera fitted with an SMC Pentax 50mm lens and 2x converter) set up on a tripod. A scale of known magnitude was placed next to, and in the same plane as, the pupil, prior to photographs being taken.

The animals were allowed to settle, and photographs were taken at f12 and 1/15 second, using ISO400 film (Kodak Gold 400). Photographs were taken 5 minutes prior to dosing of the ephedrine formulation, in order to allow determination of baseline (control) pupil diameters. The ephedrine formulation (5µl) was then administered to the eye as a pulse of charged droplets generated from the apparatus of the invention described above, and photographs taken at intervals over the next 3 hours.

Pupil diameters were determined from the devloped colour prints (approximately 15x10cm) using an electronic micrometer (Digimatic Caliper, Mitutoyo Corp., Japan). Absolute pupil diameters were established by comparing the pupil diameter with the known scale on the photographs. From these measurements, the maximum response ratio was determined from the relationship:

$$RR_{max} = \frac{\text{(pupil diameter at time t) - (average pupil diameter at time } t_0)}{\text{(average pupil diameter at time } t_0)}$$

The following results were obtained:

| RRmax | | | | | | | | |
|---|---|---|---|---|---|---|---|---|
| Animal No. | 1 | 2 | 3 | 4 | 5 | 6 | Mean | SD* |
| Time (min) | | | | | | | | |
| 0 | 0.00 | 0.00 | 0.00 | 0.00 | -0.04 | 0.00 | -0.01 | 0.01 |
| 18 | 0.09 | 0.03 | 0.11 | 0.40 | 0.17 | 0.11 | 0.15 | 0.13 |

* = standard deviation.

(continued)

| RRmax | | | | | | | | |
|---|---|---|---|---|---|---|---|---|
| Animal No. | 1 | 2 | 3 | 4 | 5 | 6 | Mean | SD* |
| 38 | 0.24 | -0.15 | 0.15 | 0.48 | 0.13 | 0.11 | 0.16 | 0.20 |
| 59 | 0.35 | 0.29 | 0.09 | 0.52 | 0.15 | 0.15 | 0.25 | 0.16 |
| 80 | 0.14 | -0.05 | 0.03 | 0.18 | -0.16 | 0.10 | 0.04 | 0.13 |
| 102 | 0.11 | -0.07 | 0.01 | 0.15 | 0.02 | 0.02 | 0.04 | 0.08 |
| 120 | 0.13 | -0.06 | 0.13 | 0.01 | 0.05 | -0.03 | 0.04 | 0.08 |
| 140 | 0.11 | -0.11 | 0.02 | 0.25 | -0.02 | - | 0.05 | 0.13 |
| 160 | 0.10 | -0.12 | 0.11 | 0.15 | -0.14 | -0.08 | 0.00 | 0.13 |
| 180 | 0.04 | -0.14 | -0.02 | 0.26 | -0.11 | -0.12 | -0.02 | 0.14 |

\* = standard deviation.

These results are presented graphically in Figure 2, and show a marked mydriatic response in the measurements taken at 18, 38 and 59 minutes in all animals, and a continuing effect up to 80 minutes in some animals, after the administration of only 5µl of the ephedrine formulation by the apparatus of this invention, which is a much lower volume than that normally required with conventional modes of application.

**Claims**

1. Spraying apparatus suitable for dispensing a liquid formulation to the eye comprising:

    (i) at least one spray nozzle (12) having an outlet (3) of sufficiently small cross section to be capable of retaining an appropriate amount of a liquid formulation, by surface tension;
    (ii) means to supply an appropriate measured volume of a liquid formulation to the spray nozzle;
    (iii) means (6) to eject a measured volume of liquid formulation from the spray nozzle as a jet;
    (iv) a piezoelectric or electromagnetic transducer (7) for exciting the jet of liquid formulation emitted from the spray nozzle to form a stream of droplets of liquid formulation;
    (v) a charging electrode (13) spaced co-axially in front of the spray nozzle and so spaced that the stream of droplets, immediately they are formed, are within the charging field of the electrode ; and
    (vi) means (10) for applying a voltage to the electrode ;

    wherein at least the spray nozzle is suitable to be hand held when in use.

2. Apparatus as claimed in claim 1 wherein the measured volume of the formulation is supplied to the nozzle by a metered valve or syringe pump.

3. Apparatus as claimed in claim 1 wherein the measured volume of the formulation is supplied by drawing into the nozzle the required amount from an external source by pipette action.

4. Apparatus as claimed in claim 1 where the measured volume of the formulation is contained in a demountable spray nozzle which is locatable on an appropriate receiving member in the apparatus.

5. Apparatus as claimed in claim 1 wherein the piezoelectric or electromagnetic transducer is adapted to excite the liquid jet formulation at a frequency of 1-200kHz.

6. Apparatus as claimed in claim 1 wherein the charging electrode is in the form of a cylinder or annulus charged to a voltage of between 0.1 and 1000v.

7. Apparatus as claimed in claim 1 comprising:

    a body member (1) of a suitable size to be hand-held, having, mounted on one wall thereof, a guide (2) in the form of a short tube, the diameter of which approximates to the size of the eye to be treated, and, positioned in the portion of the wall of the body member (1) which lies within the guide tube (2) the outlet (3) of a narrow bore tube (4) which at its other end broadens to provide a wider bore section (5) positioned in a side wall of the body membe (1), in which a spring-loaded piston (6) can operate;

a piezoelectric or electromagnetic transducer (7), located in the wall of the body member (1) and disposed in an annular manner around the outlet (3), and powered through electrical connections (8) and (9) from a voltage generator (10) via a frequency control unit (11), both of which are housed within the body member;

a demountable nozzle (12) which is capable of being detached from the apparatus, in order to be charged with the formulation to be administered, and then re-attached securely within the central orifice of the transducer (7) by a tight push fit to cooperate with the outlet (3);

and within the guide tube (2), a charging electrode (13) which is electrically insulated from the other parts of the apparatus, which is located co-axially with the transducer (7)and separated therefrom by a short distance, and which is charged to a suitable positive or negative potential through an electrical connection (14) from the voltage generator (10), its pulse frequency being controlled through the frequency control unit (11).

8. Apparatus as claimed in claim 1 wherein the liquid formulation comprises an opthalmologically acceptable liquid and optionally an opthalmologically active substance and has a viscosity in the range $10^{-3}$ to 1.0 Pas and a resistivity lower than $10^4$ ohm.cm.

## Patentansprüche

1. Sprühvorrichtung, die sich eignet, um eine flüssige Formulierung zu dem Auge zu liefern, umfassend:

   (i) mindestens eine Spraydüse (12) mit einem Auslaß (3) mit einem Querschnitt, der klein genug ist, daß er eine entsprechende Menge einer flüssigen Formulierung durch Oberflächenspannung zurückhalten kann;
   (ii) ein Mittel, um der Spraydüse ein entsprechendes gemessenes Volumen einer flüssigen Formulierung zuzuführen;
   (iii) ein Mittel (6), um ein gemessenes Volumen einer flüssigen Formulierung von der Spraydüse als Strahl auszustoßen;
   (iv) einen piezoelektrischen oder elektromagnetischen Wandler (7), um den von der Spraydüse ausgestoßenen Strahl aus flüssiger Formulierung zur Bildung eines Stroms von Tröpfchen der flüssigen Formulierung anzuregen;
   (v) einer koaxial vor der Spraydüse beabstandete Ladeelektrode (13), die derart beabstandet ist, daß der Strom von Tröpfchen sich sofort bei seiner Bildung in dem Ladefeld der Elektrode befindet; und
   (vi) ein Mittel (10) zum Anlegen einer Spannung an die Elektrode;

   wobei sich mindestens die Spraydüse dazu eignet, bei Benutzung in der Hand gehalten zu werden.

2. Vorrichtung nach Anspruch 1, bei der das gemessene Volumen der Formulierung der Düse durch ein Dosierventil oder durch eine Spritzenpumpe zugeführt wird.

3. Vorrichtung nach Anspruch 1, bei der das gemessene Volumen der Formulierung der Düse dadurch zugeführt wird, daß die erforderliche Menge von einer externen Quelle durch einen Pipettiereffekt in die Düse gezogen wird.

4. Vorrichtung nach Anspruch 1, bei der das gemessene Volumen der Formulierung in einer abnehmbaren Spraydüse enthalten ist, die an einem entsprechenden Aufnahmeglied in der Vorrichtung angeordnet werden kann.

5. Vorrichtung nach Anspruch 1, bei der der piezoelektrische oder elektromagnetische Wandler dafür ausgelegt ist, die flüssige Strahlformulierung mit einer Frequenz von 1-200 kHz anzuregen.

6. Vorrichtung nach Anspruch 1, bei der die Ladeelektrode in Form eines auf eine Spannung zwischen 0,1 und 1000 Volt aufgeladenen Zylinders oder Rings vorliegt.

7. Vorrichtung nach Anspruch 1, die folgendes umfaßt:

   ein Körperglied (1) mit einer Größe, so daß es in der Hand gehalten werden kann, das folgendes aufweist:

eine an einer Wand davon angebrachte Führung (2) in Form eines kurzen Rohres, deren Durchmesser der Größe des zu behandelnden Auges nahekommt, und, in demjenigen Teil der Wand des Körperglieds (1) positioniert ist, das im Führungsrohr (2) liegt, der Auslaß (3) eines Rohrs (4) mit kleinem Bohrungsdurchmesser, das sich an seinem anderen Ende verbreitet, um einen Abschnitt (5) mit größerem Bohrungsdurchmesser zu liefern, der in einer Seitenwand des Körperglieds (1) positioniert ist, in dem ein federbeaufschlagter Kolben (6) arbeiten kann;

einen piezoelektrischen oder elektromagnetischen Wandler (7), der in der Wand des Körperglieds (1) liegt und auf ringförmige Weise um den Auslaß (3) herum angeordnet ist und durch elektrische Verbindungen (8) und (9) von einem Spannungsgenerator (10) über eine Frequenzsteuereinheit (11), die beide in dem Gehäuseglied angeordnet sind, betrieben wird;

eine abmontierbare Düse (12), die von der Vorrichtung abgenommen werden kann, um mit der zu verabreichenden Formulierung gefüllt und dann wieder in der mittleren Öffnung des Wandlers (7) durch eine dichte Druckpassung zum Zusammenwirken mit dem Auslaß (3) sicher angebracht zu werden;

und innerhalb des Führungsrohrs (2) eine Ladeelektrode (13), die von den anderen Teilen der Vorrichtung elektrisch isoliert ist, koaxial mit dem Wandler (7) liegt und davon durch einen kurzen Abstand entfernt ist und die durch eine elektrische Verbindung (14) von dem Spannungsgenerator (10), dessen Impulsfrequenz durch die Frequenzsteuereinheit (11) gesteuert wird, auf ein geeignetes positives oder negatives Potential aufgeladen wird.

8. Vorrichtung nach Anspruch 1, bei der die flüssige Formulierung eine ophthalmologisch umbedenkliche Flüssigkeit und gegebenenfalls eine ophthalmologisch wirksame Substanz umfaßt und eine Viskosität im Bereich $10^{-3}$ bis 1,0 Pas und einen spezifischen Widerstand unter $10^4$ Ohm.cm aufweist.

**Revendications**

1. Appareil de pulvérisation convenant à la distribution d'une formulation liquide dans l'oeil, comprenant :

(i) au moins une buse de pulvérisation (12) ayant une sortie (3) de coupe transversale suffisamment petite pour être à même de retenir une quantité appropriée d'une formulation liquide sous l'effet de la tension superficielle;

(ii) un moyen pour délivrer un volume mesuré approprié de formulation liquide à la buse de pulvérisation;

(iii) un moyen (6) pour éjecter un volume mesuré de formulation liquide de la buse de pulvérisation sous la forme d'un jet;

(iv) un transducteur piézoélectrique ou électromagnétique (7) pour exciter le jet de formulation liquide émis par la buse de pulvérisation pour former un courant de gouttelettes de formulation liquide;

(v) une électrode de chargement (13) espacée coaxialement devant la buse de pulvérisation et assez espacée pour que le courant de gouttelettes, dès qu'elles sont formées, se trouve dans le champ de chargement de l'électrode; et

(vi) un moyen (10) pour appliquer une tension à l'électrode;

dans lequel au moins la buse de pulvérisation convient pour être tenue à la main lors de son utilisation.

2. Appareil selon la revendication 1, dans lequel le volume mesuré de la formulation est délivré à la buse par une pompe de vanne ou de seringue dosée.

3. Appareil selon la revendication 1, dans lequel le volume mesuré de la formulation est délivré par aspiration dans la buse de la quantité requise depuis une source externe par l'action d'une pipette.

4. Appareil selon la revendication 1, dans lequel le volume mesuré de la formulation est contenu dans une buse de pulvérisation démontable qui peut être positionnée sur un élément de réception approprié dans l'appareil.

5. Appareil selon la revendication 1, dans lequel le transducteur piézoélectrique ou électromagnétique est à même d'exciter la formulation du jet liquide à une fréquence de 1-200 kHz.

6. Appareil selon la revendication 1, dans lequel l'électrode de chargement se présente sous la forme d'un cylindre ou d'un anneau chargé à une tension entre 0,1 et 1000 V.

**7.** Appareil selon la revendication 1, comprenant :

un élément formant corps (1), de taille appropriée pour être tenu à la main, sur une paroi duquel est monté un guide (2) sous la forme d'un tube court, dont le diamètre est approximativement de la taille de l'oeil à traiter et, disposée dans la partie de la paroi de l'élément formant corps (1) qui se trouve dans le tube de guidage (2), la sortie (3) d'un tube étroit alésé (4) qui, à son autre extrémité, s'élargit pour former une section alésée plus large (5) positionnée dans une paroi latérale de l'élément formant corps (1), dans laquelle un piston (6) sollicité par un ressort peut fonctionner;

un transducteur piézoélectrique ou électromagnétique (7) situé dans la paroi de l'élément formant corps (1), disposé de manière annulaire autour de la sortie (3) et activé par des connexions électriques (8) et (9) à partir d'un générateur de tension (10) via une unité de régulation de fréquence (11), tous deux logés dans l'élément formant corps;

une buse démontable (12) qui peut être détachée de l'appareil, de manière à pouvoir être chargée en formulation à administrer, puis être remise en place solidement dans l'orifice central du transducteur (7) par une pression étroite pour lui permettre de coopérer avec la sortie (3); et,

dans le tube de guidage (2), une électrode de chargement (13) qui est isolée électriquement des autres parties de l'appareil, est positionnée coaxialement avec le transducteur (7) et séparée de celui-ci par une petite distance, et chargée à un potentiel positif ou négatif approprié par une connexion électrique (14) du générateur de tension (10), sa fréquence d'impulsion étant réglée par l'unité de régulation de fréquence (11).

**8.** Appareil selon la revendication 1, dans lequel la formulation liquide comprend un liquide ophtalmologiquement acceptable et, en option, une substance ophtalmologiquement active et a une viscosité dans la plage de $10^{-3}$ à 1,0 Pas et une résistivité inférieure à $10^4$ ohms.cm.

# Fig.1.

# Fig.2.